(19) Europäisches Patentamt / European Patent Office / Office européen des brevets

(11) **EP 4 037 649 B1**

(12) **FASCICULE DE BREVET EUROPEEN**

(45) Date de publication et mention de la délivrance du brevet:
**13.03.2024 Bulletin 2024/11**

(21) Numéro de dépôt: **20792472.1**

(22) Date de dépôt: **29.09.2020**

(51) Classification Internationale des Brevets (IPC):
**A61K 8/49** *(2006.01)*   **A61Q 5/00** *(2006.01)*
**A61K 31/4418** *(2006.01)*   **A61P 17/08** *(2006.01)*

(52) Classification Coopérative des Brevets (CPC):
**A61Q 5/006; A61K 8/49; A61K 8/4926; A61K 31/4418**

(86) Numéro de dépôt international:
**PCT/FR2020/051698**

(87) Numéro de publication internationale:
**WO 2021/064316 (08.04.2021 Gazette 2021/14)**

(54) **ASSOCIATION DE CICLOPIROXOLAMINE ET PIROCTONE OLAMINE POUR COMBATTRE LES PELLICULES**

KOMBINATION AUS CICLOPIROXOLAMIN UND PIROCTON-OLAMIN ZUR BEKÄMPFUNG VON SCHUPPEN

COMBINATION OF CICLOPIROXOLAMINE AND PIROCTONE OLAMINE TO FIGHT DANDRUFF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.09.2019 FR 1910808**

(43) Date de publication de la demande:
**10.08.2022 Bulletin 2022/32**

(73) Titulaire: **Pierre Fabre Dermo-Cosmétique 81500 Lavaur (FR)**

(72) Inventeurs:
• **LUC, Joëlle**
  **31150 FENOUILLET (FR)**
• **MICHEL, Carole**
  **31460 MOURVILLES-BASSES (FR)**

(74) Mandataire: **Regimbeau**
  **20, rue de Chazelles**
  **75847 Paris Cedex 17 (FR)**

(56) Documents cités:
**WO-A1-2018/083675    WO-A2-2009/108713
WO-A2-2009/112470    US-A1- 2019 142 732**

• **DATABASE GNPD [Online] MINTEL; 21 janvier 2019 (2019-01-21), anonymous: "Anti-Dandruff Shampoo", XP055699461, extrait de www.gnpd.com Database accession no. 6279545**
• **DATABASE GNPD [Online] MINTEL; 2 juillet 2015 (2015-07-02), anonymous: "Shampoo for Severe Dandruff Conditions with Itching", XP055708126, extrait de www.gnpd.com Database accession no. 3276083**
• **CHRISTINE ROQUES ET AL: "In vitro antifungal efficacy of ciclopirox olamine alone and associated with zinc pyrithione compared to ketoconazole against Malassezia globosa and Malassezia restricta reference strains", MYCOPATHOLOGIA, KLUWER ACADEMIC PUBLISHERS, DO, vol. 162, no. 6, 1 décembre 2006 (2006-12-01), - 1 décembre 2006 (2006-12-01), pages 395-400, XP019452935, ISSN: 1573-0832, DOI: 10.1007/S11046-006-0075-0**
• **DATABASE GNPD [Online] MINTEL; 21 January 2019 (2019-01-21), anonymous: "Anti-Dandruff Shampoo", XP055699461, Database accession no. 6279545**
• **DATABASE GNPD [Online] MINTEL; 2 July 2015 (2015-07-02), anonymous: "Shampoo for Severe Dandruff Conditions with Itching", XP055708126, Database accession no. 3276083**

**Description**

**DOMAINE TECHNIQUE DE L'INVENTION**

La présente invention concerne une association de Ciclopiroxolamine et de Piroctone Olamine et les compositions contenant cette association pour leurs applications dans les domaines de la cosmétique et de la dermatologie pour lutter contre les pellicules.

**ETAT DE LA TECHNIQUE**

Les pellicules touchent une personne sur deux en France, leur présence est bénigne, mais elles donnent un aspect inesthétique.

Les pellicules sont des squames de peau, et plus précisément des cellules superficielles du cuir chevelu ayant desquamé, mais d'une taille inhabituellement grande, plus épaisses ou plus nombreuses que les cellules normales. Le cuir chevelu, comme toute la peau, se renouvelle normalement en éliminant des cellules superficielles de l'épiderme. Les pellicules sont dites sèches ou grasses.

Les pellicules sèches sont visibles à l'oeil nu et se présentent comme des parcelles de la couche cornée du cuir chevelu. Elles sont blanches, plates et se détachent spontanément. Elles sont souvent accompagnées de démangeaisons désagréables. Elles vont généralement de pair avec un cuir chevelu sec.

Les pellicules grasses sont plutôt petites, rondes, jaunâtres et grasses. Elles adhèrent au cuir chevelu et aux cheveux. Elles y forment une couche jaunâtre et collante. Elles sont accompagnées de démangeaisons. En général, elles sont la conséquence d'une séborrhée et donc souvent associées à un cuir chevelu et à des cheveux gras.

Les pellicules sont un trouble de la formation de l'épiderme. Un cuir chevelu en bonne santé présente des cellules mortes tous les jours sans qu'on s'en aperçoive. Ce renouvellement cellulaire s'établit selon un cycle régulier d'environ 28 jours. Durant ce processus, les cellules de l'épiderme ont le temps de finaliser correctement leur maturation, de se désolidariser progressivement les unes des autres et une fois arrivées à la surface, de se détacher une à une de façon invisible. Dans le cas de pellicules, la division des cellules de la couche basale de l'épiderme est trop rapide sous l'action d'une inflammation du cuir chevelu. Le renouvellement cellulaire s'effectue alors en 5 à 14 jours. Les cellules qui montent vers la surface de la peau n'ont pas le temps de perdre leur eau et de se durcir. Elles atteignent la surface sans être suffisamment desséchées, ce qui forme des amas de cellules sur le cuir chevelu, qui deviennent visibles : ce sont les pellicules.

Il existe deux états pelliculaires selon la sévérité :

- Les pellicules légères et discrètes : ce sont des petits squames blanchâtres correspondant à des amas de cellules mortes très volatiles qui tombent du cuir chevelu et viennent parsemer les vêtements ;
- La dermite séborrhéique : il s'agit de pellicules associées à une forte inflammation du cuir chevelu induisant des rougeurs et des démangeaisons.

La dermatite séborrhéique est en fait une dermatose inflammatoire cutanée fréquente (observée dans environ 3 % de la population) se présentant sous la forme de plaques rouges, recouvertes de squames grasses et jaunâtres, plus ou moins prurigineuses, prédominant dans les zones riches en glandes sébacées, les zones séborrhéiques.

L'épiderme du cuir chevelu est recouvert d'un film lipidique appelé sébum dont le rôle est, entre autres, de le protéger contre le dessèchement. *Malassezia* parfois aussi appelé *Pityrosporum ovale,* est le nom d'une levure appartenant au groupe des *Fungi imperfecti* et faisant partie de la flore commensale des humains. Les *Malassezia* (*M.*) sont connues de longue date en pathologie humaine. On distingue plusieurs espèces impliquées dans ces pathologies : *M. furfur, M. sympodialis, M. globosa, M. restricta, M. obtusa.* Les *Malassezia* sont des levures lipophiles et kératinophiles, elles sont lipodépendantes. Il est établi que ce champignon est un agent faiblement pathogène pour l'homme. M. furfur est présente chez pratiquement 100% de la population. Cependant, il est également établi que *M. furfur,* est responsable chez environ 3% de la population de dermatoses telles que la dermite séborrhéique de l'adulte. Chez les sujets sains, les espèces les plus fréquemment rencontrées sont *M. sympodialis, M. globosa* et *M. restricta,* quels que soient les milieux de culture utilisés, la méthode de collecte de l'échantillon ou le pays de l'étude, *M. restricta* semble prédominer sur le cuir chevelu, tandis que *M. sympodialis* prédomine sur le tronc et *M. globosa* est largement répandue et distribuée de manière égale sur les zones séborrhéiques. Malassezia se développe aisément dans des milieux gras, comme le cuir chevelu où le sébum est sécrété en quantité particulièrement élevée créant donc un environnement favorable pour son développement. Ce champignon peut « digérer » des lipides présents dans le sébum et produire des acides gras particulièrement irritants pour la peau et le cuir chevelu. Dans la dermatite séborrhéique, le mécanisme pathogénique exact reste peu clair, mais plusieurs facteurs permettent d'affirmer que *Malassezia* joue un rôle prépondérant. En effet il a été démontré que : a) la proportion de Malassezia est plus importante chez les personnes atteintes de dermatite séborrhéique, ainsi que sur la peau atteinte par rapport à la peau saine chez les patients souffrant de dermatite séborrhéique (Tajima et al., J Invest Dermatol, 2008, 128, 345-351) et b) les traitements antifongiques diminuent efficacement la symptomalotolgie (Piérard-Franchimont et al., Dermatology, 2001, 202, 171-176). L'hypothèse actuelle soutient que la dermatite séborrhéique ne serait pas causée par une croissance exagérée de *Malassezia,* mais par une réponse anormale de l'hôte à ces champignons. Une hypothèse avance que ces levures produiraient une lipase, qui aurait pour action de transformer les triglycérides du sébum en acides gras libres, qui eux-mêmes induiraient une inflammation. Une étude récente a en effet mis en évidence que l'espèce *M. globosa* possédait un gène, LP1, dont l'expression permet de produire une lipase (DeAngelis et al., J Invest Dermatol 2007, 127, 2138-2146).

Produits en trop grande quantité, les acides gras peuvent induire une inflammation à l'origine des pellicules. Deux espèces, *M. globosa* et *M. restricta* semblent aujourd'hui plus étroitement impliquées dans l'état pelliculaire. Pour environ 50% des individus, sébum et *Malassezia* coexistent en bonne harmonie. Pour les 50% restants, un déséquilibre peut survenir, plusieurs facteurs entrant en ligne de compte. Tout le monde ne sécrète pas la même quantité de sébum. Certaines personnes en sécrètent une quantité suffisamment importante pour être propice au développement excessif de *Malassezia* et donc à la formation de pellicules. Par ailleurs, la quantité de sébum est variable au cours du temps, notamment selon les sécrétions hormonales. Enfin d'autres facteurs peuvent favoriser l'apparition des pellicules, comme le stress, la fatigue, la pollution, le manque de soleil, certaines maladies comme la maladie de Parkinson, certains médicaments, comme les neuroleptiques, etc.

Les pellicules apparaissent souvent à l'adolescence au moment de la puberté lorsque débute la sécrétion de sébum. Les hommes semblent plus fréquemment atteints que les femmes, sans doute sous l'effet des hormones androgènes.

Des compositions antipelliculaires, particulièrement sous forme de shampoings, sont connues et sont disponibles commercialement depuis de nombreuses années. Elles contiennent généralement des actifs chimiques comme le kétoconazole, la pyrithione de zinc, l'octopirox, la piroctone olamine, l'acide salicylique, le sulfure de sélénium et l'acide azelaïque. Certains composés, notamment la pyrithione de zinc, sont soupçonnés d'induire des problèmes environnementaux, notamment d'écotoxicité en induisant une toxicité chez les poissons. Il est donc important de trouver de nouveaux actifs ou de nouvelles combinaisons d'actifs qui soient respectueux de l'environnement, c'est-à-dire sans pyrithione de zinc, tout en étant très efficace et avec une rapidité d'action.

Ainsi, malgré les nombreuses options actuellement disponibles les consommateurs ont toujours besoin de disposer de nouveaux produits pour combattre les pellicules et/ou pour en prévenir la formation.

## RESUME DE L'INVENTION

De façon surprenante, les inventeurs ont découvert que l'association de Ciclopiroxolamine et de Piroctone Olamine présentait une activité synergique sur *Malassezia furfur* dans le modèle mis en oeuvre.

La présente invention a ainsi pour but de proposer un traitement efficace contre les pellicules ainsi que pour prévenir et/ou traiter la dermatite séborrhéique, et ce sans pyrithione de zinc. De préférence, ce traitement est effectué par voie topique, ce qui permet une meilleure efficacité.

Une caractéristique importante de l'invention est que l'association comprenant de la Ciclopiroxolamine et de la Piroctone Olamine est dénuée de pyrithione de zinc.

En effet, selon la présente invention, il faut comprendre que chaque fois qu'il est mentionné l'association de la Ciclopiroxolamine et de la Piroctone Olamine, il n'y a pas de pyrithione de zinc.

La présente invention a donc pour objet une association comprenant de la Ciclopiroxolamine et de la Piroctone Olamine, sans pyrithione de zinc pour son utilisation, de préférence topique, dans la prévention et/ou le traitement des pellicules, en particulier les pellicules légères et discrètes, et/ou pour son utilisation pour prévenir et/ou traiter la dermatite séborrhéique.

La présente invention a également pour objet l'utilisation, de préférence topique, de l'association comprenant de la Ciclopiroxolamine et de la Piroctone Olamine, sans pyrithione de zinc dans la prévention et/ou le traitement des pellicules, en particulier les pellicules légères et discrètes, et/ou dans la prévention et/ou le traitement la dermatite séborrhéique.

La présente invention a également pour objet l'utilisation de l'association comprenant de la Ciclopiroxolamine et de la Piroctone Olamine, sans pyrithione de zinc, pour la fabrication d'une composition dermatologique ou cosmétique, de préférence topique, destinée à la prévention et/ou au traitement des pellicules, en particulier les pellicules légères et discrètes.

L'invention vise ainsi l'utilisation d'une association selon l'invention pour la fabrication d'une composition dermatologique pour le traitement et/ou la prévention de la dermatite séborrhéique.

Selon un autre aspect, l'invention vise une utilisation d'une association selon l'invention pour la fabrication d'une composition dermocosmétique pour combattre les pellicules et/ou en prévenir la formation.

De manière avantageuse, l'invention vise une association comprenant, ou consistant en, ciclopiroxolamine et piroctone olamine, comme actif antipelliculaire au sein d'une composition dermatologique ou cosmétique, en particulier topique, pour la prévention ou le traitement des pellicules.

De manière tout aussi avantageuse, l'invention vise une association comprenant, ou consistant en, ciclopiroxolamine et piroctone olamine, comme actif antipelliculaire au sein d'une composition dermatologique ou cosmétique, en particulier topique, pour prévenir et/ou traiter la dermatite séborrhéique.

En particulier, l'association ciclopiroxolamine et piroctone olamine est dénuée de pyrithione de zinc et, avantageusement, ladite association est le seul ingrédient actif antipelliculaire de la composition.

La présente invention a également pour objet une méthode de prévention et/ou de traitement des pellicules comprenant l'administration, notamment par application topique, d'une quantité efficace de l'association comprenant de la Ciclopiroxolamine et de la Piroctone Olamine, sans pyrithione de zinc à une personne en ayant besoin.

En particulier il s'agit de pellicules légères et discrètes.

Dans la méthode selon l'invention, la méthode ne prévoit pas d'utilisation de pyrithione de zinc. Selon l'invention, l'association ciclopiroxolamine et piroctone olamine est avantageusement le seul agent actif antipelliculaire mis en oeuvre dans la méthode.

La présente invention vise également une méthode cosmétique de traitement et/ou de prévention des pellicules comprenant l'administration, notamment par application topique, de l'association comprenant de la Ciclopiroxolamine et de la Piroctone Olamine, sans pyrithione de zinc. En particulier il s'agit de pellicules légères et discrètes. En particulier, la méthode ne prévoit pas d'utilisation de pyrithione de zinc.

Avantageusement, selon l'invention, l'association ciclopiroxolamine et piroctone olamine est le seul agent actif antipelliculaire de la composition et de la méthode selon l'invention. Selon un autre aspect, l'invention vise encore l'association comprenant de la Ciclopiroxolamine et de la Piroctone Olamine, sans pyrithione de zinc pour son utilisation pour prévenir et/ou traiter la dermatite séborrhéique comprenant l'administration, notamment par application topique au du cuir chevelu, d'une association selon l'invention ou d'une composition selon l'invention.

## DESCRIPTION DETAILLEE DE L'INVENTION

La Ciclopiroxolamine et la Piroctone Olamine sont des substances antifongiques de la famille des hydroxy-pyridones. Ces composés sont souvent utilisés dans les produits cosmétiques en médecine humaine, par exemple la Piroctone Olamine est utilisée à une concentration maximale de 1% (produits rinçables) ou 0,05% à 0,1% (concentration suffisante dans les produits qui ne se rincent pas). Ces deux substances sont très efficaces et sont même considérées comme des agents antipelliculaires de seconde génération. De plus, elles possèdent une très faible toxicité. Elles sont par ailleurs particulièrement adaptées à la fabrication de shampoings antipelliculaires et de soins pour les cheveux de par leur solubilité dans les tensio-actifs aqueux ou mixtes (eau-alcool). Les hydroxy-pyridones possèdent des propriétés antifongiques mais également antibactériennes et montrent d'excellentes capacités de diffusion dans la kératine.

La Piroctone Olamine est la dénomination courante de ce composé. On la retrouve dans la littérature sous le terme d'Octopirox. Elle se présente sous forme de sel, combiné à de l'éthanolamine, ce qui lui vaut également l'appellation de sel de Piroctone ethanolamine. Son numéro CAS est le 68890-66-4.

La ciclopiroxolamine appelée encore ciclopirox olamine a comme numéro CAS le 29342-05-0.

L'action antipelliculaire de ces deux substances est attribuée à leurs actions antibactériennes et antifongiques. L'action antifongique de la famille des hydroxy-pyridones a été étudiée à partir du modèle de la ciclopiroxolamine. Etant donné que son activité antifongique a été attribuée au groupement hydroxy-pyridone, on peut extrapoler ce mode d'action à toute la famille. Ce mode d'action est complexe et partiellement compris. L'activité antifongique semble résulter de multiples processus métaboliques au sein de la cellule visée. Les hydroxy-pyridones ont une haute affinité pour les cations métalliques trivalents comme le fer et l'aluminium. Or ce cofacteur enzymatique est nécessaire aux cytochromes mitochondriaux qui sont impliqués dans le transport des électrons, en particulier au complexe I (NADH-Ubiquinone oxydoreductase). La neutralisation du fer et de l'aluminium inhibe cette réaction métabolique et donc la production d'énergie cellulaire. Par ailleurs, le Ciclopirox inhibe l'activité des catalases et des peroxydases intracellulaires chargées de dégrader les peroxydes toxiques pour la cellule. Des études ont également montré que le ciclopirox altère les mécanismes de transport transmembranaire de la levure. La capture des nutriments vers l'intérieur des micro-organismes est donc affectée. Dans les cellules en croissance, la déplétion en acides aminés essentiels et nucléotides conduit à une diminution de la synthèse protéique et des acides nucléiques. La Piroctone Olamine présente aussi une action inhibitrice sur la réplication de l'ADN. Cette hydroxy-pyridone n'a pas d'effet direct sur les enzymes de la réplication mais la chute du taux d'ATP rend cette synthèse impossible. La ciclopiroxolamine a également des effets anti-inflammatoires en inhibant la 5-lipoxygenase et la cyclo-oxygenase. Etant donné la complexité du mode d'action des hydroxy-pyridones, il est peu probable que des phénomènes de résistance apparaissent.

L'utilisation de la Ciclopiroxolamine ou de la Piroctone Olamine est assez fréquente et on retrouve l'un ou l'autre dans de nombreux produits, parfois associés à d'autres agents antimicrobiens notamment la pyrithione de zinc. On peut citer par exemple le shampoing dermatologique antipelliculaire Dercos® comprenant de la Piroctone Olamine et de l'acide salicylique, le shampoing antipelliculaire Hegor® contenant de la Piroctone Olamine et de la pyrithione de zinc, ou encore le shampoing traitant la dermatite séborrhéique du cuir chevelu avec le Ciclopirox (Sébiprox®) ou encore (STIPROX®). En revanche à ce jour, aucun produit sans pyrithione de zinc, n'associe la Ciclopiroxolamine à la Piroctone Olamine.

Composition dermatologique ou cosmétique

La présente invention concerne aussi une composition, en particulier une composition dermatologique ou cosmétique (e.g. dermo-cosmétique), pour son utilisation pour son utilisation pour prévenir et/ou traiter la dermatite séborrhéique ou pour combattre les pellicules et/ou en prévenir la formation, en particulier les pellicules légères et discrètes, comprenant l'association de la Ciclopiroxolamine et de la Piroctone Olamine, telle que définie ci-dessus, avec un excipient dermatologiquement ou cosmétiquement acceptable, plus particulièrement acceptable pour une application topique.

La composition est ainsi dénuée de pyrithione de zinc. Avantageusement l'association constituée de Ciclopiroxolamine et de Piroctone Olamine représente le seul actif antipelliculaire de la composition.

L'invention vise aussi une composition comprenant à titre de principe actif une association comprenant de la Ciclopiroxolamine et de la Piroctone Olamine, sans pyrithione de zinc avec au moins un excipient dermatologiquement ou cosmétiquement acceptable pour son utilisation pour prévenir et/ou traiter la dermatite séborrhéique. L'invention vise ainsi une composition comprenant à titre de principe actif une association comprenant de la Ciclopiroxolamine et de la Piroctone Olamine, sans pyrithione de zinc avec au moins un excipient dermatologiquement ou cosmétiquement acceptable pour son utilisation pour combattre les pellicules et/ou en prévenir la formation.

La combinaison de ces deux actifs présente une activité antipelliculaire très intéressante, comme démontré dans les exemples de la présente demande, et ce par son niveau d'efficacité mais également par sa rapidité d'action. Une telle rapidité dans l'effet inhibiteur des souches *M. furfur,* présente un effet indubitablement avantageux, et surprenant, permettant ainsi un temps de pose réduit et raisonnable dans le cadre de traitement antipelliculaire topique.

La combinaison selon l'invention a aussi une action très favorable sur la dermite séborrhéique tel que démontré via son action sur *M. globosa* impliqué dans la dermatite séborrhéique.

La présente invention concerne ainsi un agent de traitement capillaire comprenant, ou consistant en, une association Ciclopiroxolamine et Piroctone Olamine, pour son utilisation pour le traitement ou la prévention des états pelliculaires. L'agent de traitement capillaire selon l'invention est dénué de pyrithione de zinc. Dans la présente description l'association Ciclopiroxolamine et Piroctone Olamine est assimilée à un agent de traitement capillaire. La présente invention concerne ainsi un agent de traitement capillaire comprenant, ou consistant en, une association Ciclopiroxolamine et Piroctone Olamine, pour son utilisation pour le traitement ou la prévention de la dermite séborrhéique. L'agent de traitement capillaire selon l'invention est dénué de pyrithione de zinc. Dans la présente description l'association Ciclopiroxolamine et Piroctone Olamine est assimilée à un agent de traitement capillaire.

L'invention vise aussi l'utilisation d'un agent de traitement capillaire comprenant, ou consistant en, une association Ciclopiroxolamine et Piroctone Olamine, pour le traitement ou la prévention des états pelliculaires. L'agent de traitement capillaire selon l'invention est dénué de pyrithione de zinc.

L'invention concerne aussi un procédé de préparation d'une composition antipelliculaire pour application topique sur le cuir chevelu et/ou les cheveux, la composition comprenant une association Ciclopiroxolamine et Piroctone Olamine en tant qu'agent antipelliculaire actif, le procédé comprenant les étapes consistant à: a) mélanger une association Ciclopiroxolamine et Piroctone Olamine avec un véhicule dermatologiquement ou cosmétiquement acceptable; et b) emballer le mélange résultant de l'étape (a) dans un emballage approprié. De manière avantageuse, le procédé de fabrication de la composition ne prévoit aucun ajout de pyrithione de zinc.

La présente invention a également pour objet l'utilisation, de préférence topique, d'une composition dermatologique ou cosmétique (e.g. dermo-cosmétique) comprenant l'association de la Ciclopiroxolamine et de la Piroctone, telle que définie ci-dessus, avec un excipient dermatologiquement ou cosmétiquement acceptable, plus particulièrement acceptable pour une application topique, dans la prévention et/ou le traitement des pellicules, en particulier des pellicules légères et discrètes.

La présente invention a également pour objet l'utilisation, de préférence topique, d'une composition dermatologique comprenant l'association de la Ciclopiroxolamine et de la Piroctone, telle que définie ci-dessus, avec un excipient dermatologiquement acceptable, plus particulièrement acceptable pour une application topique, dans la prévention et/ou le traitement de la dermite séborrhéique.

La présente invention a également pour objet une méthode de prévention et/ou de traitement des pellicules comprenant l'administration, notamment par application topique, d'une quantité efficace d'une composition dermatologique ou cosmétique (e.g. dermo-cosmétique) comprenant l'association de la Ciclopiroxolamine et de la Piroctone, telle que définie ci-dessus, avec un excipient dermatologiquement ou cosmétiquement acceptable, plus particulièrement acceptable pour une application topique, à une personne en ayant besoin. Comme indiqué, avantageusement, l'association Ciclopiroxolamine et Piroctone Olamine est le seul actif antipelliculaire utilisé dans la méthode selon l'invention. L'invention vise ainsi une méthode cosmétique de traitement et/ou de prévention des pellicules, en particulier des pellicules légères et discrètes, comprenant l'administration, notamment par application topique au niveau des cheveux ou du cuir chevelu, d'une association selon l'invention ou d'une composition selon l'invention.

La présente invention vise également une méthode cosmétique de traitement et/ou de prévention des pellicules, en particulier de pellicules légères et discrètes, comprenant l'administration, notamment par application topique, d'une composition dermatologique ou cosmétique (e.g. dermo-cosmétique) comprenant l'association de la Ciclopiroxolamine et de la Piroctone, telle que définie ci-dessus, avec un excipient dermatologiquement ou cosmétiquement acceptable, plus particulièrement acceptable pour une application topique. Comme indiqué, avantageusement, l'association Ciclopiroxolamine et Piroctone Olamine est le seul actif antipelliculaire utilisé dans la méthode selon l'invention.

Dans la présente invention, on entend désigner par « dermatologiquement ou cosmétiquement acceptable » ce qui est utile dans la préparation d'une composition dermatologique ou cosmétique, qui est généralement sûre, non toxique et ni biologiquement ni autrement non souhaitable et qui est acceptable pour une utilisation thérapeutique ou cosmétique, notamment par application topique.

La présente invention concerne aussi un procédé permettant d'obtenir une efficacité antipelliculaire sur cheveux ou cuir chevelu comprenant les étapes consistant à : (i) mouiller les cheveux avec de l'eau ; (ii) appliquer une quantité efficace d'une composition antipelliculaire selon la présente invention sur les cheveux ; (iii) rincer la composition antipelliculaire des cheveux avec de l'eau ; et (iv) répéter éventuellement les étapes (ii) et (iii).

La présente invention vise encore un procédé pour tuer ou retarder la croissance de *Malassezia* spp., en particulier *M. globosa et/ou M. restricta,* le procédé comprenant l'étape consistant à mettre en contact Malassezia spp., en particulier *M. globosa et/ou M. restricta* avec une composition selon l'invention efficace pour tuer ou retarder la croissance de Malassezia spp., en particulier *M. globosa et/ou M. restricta.*

Selon un mode de réalisation, l'invention vise encore un procédé permettant d'obtenir une efficacité anti-séborrhéique sur la peau, comprenant les étapes consistant à - (i) optionnellement mouiller la peau avec de l'eau, - (ii) appliquer une quantité efficace d'une composition anti-séborrhéique telle que décrite dans l'une des revendications 3 à 7 sur la peau, - (iii) rincer la composition anti-séborrhéique de la peau avec de l'eau, - (iv) éventuellement répéter les étapes (ii) et (iii).

Dans un mode de réalisation particulier, les compositions dermatologiques ou cosmétiques selon l'invention comprennent au moins un autre principe actif antipelliculaire, différent de la pyrithione de zinc, et en particulier un antifongique, excepté la pyrithione de zinc. De manière avantageuse le au moins un autre principe actif antipelliculaire, autre que la pyrithione de zinc, est choisi dans le groupe constitué par la pseurotine A, le kétoconazole, le miconazole, l'acide salicylique, le sulfure de sélénium, le goudron de houille, l'acide azélaïque, le climbazole, l'acide undécylénique et leurs mélanges.

L'invention vise de préférence des compositions, en particulier dermatologiques ou cosmétiques, se présentant sous une forme propre et adaptée à une application topique, en particulier au niveau des cheveux et/ou du cuir chevelu.

Les compositions dermatologiques ou cosmétiques selon l'invention pourront se présenter ainsi sous les formes qui sont habituellement connues pour une administration topique, c'est-à-dire notamment les lotions, les shampoings, les shampoings secs, les après-shampoings, les baumes, les mousses, les gels, les dispersions, les émulsions, les sprays, les sérums, les masques, les crèmes, les patchs.

Avantageusement, les compositions selon l'invention peuvent se présenter sous les formes qui sont habituellement connues pour une administration topique sur les cheveux et le cuir chevelu, c'est-à-dire notamment un shampoing, un shampoing sec, un après-shampoing, une crème capillaire, une lotion capillaire, un masque ou un spray, en particulier un spray sans rinçage.

Il est ainsi distingué des produits formulés pouvant être rincés et des produits formulés ne nécessitant pas de rinçage.

Ces compositions contiennent généralement, outre l'association de la Ciclopiroxolamine et de la Piroctone Olamine selon la présente invention, un milieu physiologiquement acceptable, en général à base d'eau ou de solvant, par exemple des alcools, des éthers ou des glycols. Elles peuvent également contenir des agents tensioactifs, des agents complexants, des conservateurs, des agents stabilisants, des émulsifiants, des épaississants, des gélifiants, des humectants, des émollients, des oligo-éléments, des huiles essentielles, des parfums, des colorants, des agents hydratants ou des eaux thermales, etc.

Avantageusement, les compositions selon la présente invention comprennent 0,01 à 2% en poids, de préférence 0,1 à 2% en poids, de manière encore préférée 0,5 à 2% en poids de Ciclopiroxolamine par rapport au poids total de la composition. D'une manière préférée la composition comprend 1,5% en poids de Ciclopiroxolamine par rapport au poids total de la composition.

Avantageusement, les compositions selon la présente invention comprennent 0,01 à 1% en poids, de préférence 0,05 à 1% en poids, de manière encore préférée 0,1 à 1% en poids de Piroctone Olamine par rapport au poids total de la composition. D'une manière préférée la composition comprend 0,3% en poids de Piroctone Olamine par rapport au poids total de la composition.

Les compositions de l'invention comprennent ainsi, avantageusement, en % en poids de la composition finale, de 0,05 à 1% de Piroctone Olamine et de 0,1 à 2% de Ciclopiroxolamine. Particulièrement, une composition selon l'invention comprend, en % en poids de la composition, environ 0,3 % de Pirocotone Olamine et environ 1,5% de Ciclopiroxolamine. Particulièrement encore, une composition selon l'invention comprend, en % en poids de la composition, environ 0,15 % de Pirocotone Olamine et environ 0,75% de Ciclopiroxolamine.

Les compositions selon la présente invention comprennent de la Ciclopiroxolamine et de la Piroctone Olamine dans un ratio massique Ciclopiroxolamine : Piroctone Olamine compris entre 1 : 10 et 10 : 1, plus particulièrement compris entre 1 : 5 et 5 : 1.

De manière particulière, lorsque l'on exprime les quantités de Ciclopiroxolamine et de Piroctone Olamine dans l'association selon l'invention, en parts en poids, cette association comprend entre 0,5 et 2 parts de Piroctone Olamine et avantageusement entre 1 et 10 parts de Ciclopiroxolamine. De manière préférée, l'association selon l'invention comprend, en parts en poids, 1 part de Piroctone Olamine et 5 parts de Ciclopiroxolamine.

De telles compositions peuvent être fabriquées selon des procédés bien connus de l'homme du métier.

Les exemples qui suivent illustrent l'invention sans en limiter la portée.

EXEMPLES

Exemple 1 : Effets de l'association de la Ciclopiroxolamine et de la Piroctone Olamine sur l'inhibition de la croissance des colonies de *Malassezia globosa et restricta*

Les souches sont issues de la collection de l'Institut Pasteur (Collection de champignons, Paris France) pour *M. globosa* (IP2387.96) et de la collection de CBS-KNAW pour *M. restricta (CBS7877)*. Ces souches représentent et correspondent à la majorité de souches impliquées dans les pellicules. Les souches sont incubées pendant 5 jours at 30°C sur de la gélose de Dixon modifié sous des conditions aérobies. Les suspensions d'inoculation sont préparées dans de l'eau distillée stérile, et sont concentrées à $10^7$ UFC/ml. La détermination des Concentrations Minimales Inhibitrices (CMI) est réalisée par microméthode en milieu liquide. 100$\mu$l de milieu de culture liquide sont déposés dans chaque puits d'une microplaque stérile 96 puits. 100$\mu$l des solutions à tester sont déposés dans le premier puits d'une ligne de la plaque. Des dilutions de raison 2 sont ensuite réalisées des puits 1 à 10. Les suspensions-test de chaque microorganisme sont préparées extemporanément dans du Tryptone sel. 100$\mu$l sont déposés dans chaque puits d'une seconde microplaque, à l'exception de la colonne 11. L'ensemble de la première plaque est inoculée, à l'aide d'un ensemenceur multipoint Denley, à partir de la seconde microplaque. Après incubation les CMI sont définies comme la plus grande dilution avec absence de croissance visible. Les colonnes 11 et 12 servent respectivement de témoin négatif et positif de croissance.

Les produits testés sont :

- La Ciclopiroxolamine, à 3% (P/P) avec de l'eau distillée stérile,
- La Piroctone Olamine à 0,3% (P/P) avec 10% d'éthanol,
- Le Ketoconazole (témoin positif) à 1000$\mu$g/ml (P/P) avec 10% de DMSO,
- L'association Piroctone Olamine / Ciclopiroxolamine, aux concentrations respectivement de 0,15% et 0,75%.

Les solutions sont préparées au moment de l'essai et utilisées immédiatement. Un témoin éthanol a été réalisé, solution à 10%.

La détermination des Concentrations Minimales Fongicides (CMF) est réalisée par repiquage des microplaques de CMI sur milieu gélosé, à l'aide de l'ensemenceur multipoint Denley. Après incubation, la CMF est définie comme la plus grande dilution avec absence de croissance visible. Tous les essais sont réalisés en duplicate.

L'effet synergique de deux produits est déterminé par le calcul de l'indice de synergie (FIC, Fractional Inhibitory Concentration).

$$\text{FIC Index} = (\text{CMI produit A associé}) / (\text{CMI produit A seul}) + (\text{CMI produit B associé}) / (\text{CMI produit B seul}).$$

Une synergie est révélée lorsque la valeur du FIC index est < 0,5.

Résultats

Les tableaux 1 (*Malassezia globosa*) et 2 (*Malassezia restricta*) ci-dessous indiquent les valeurs de CMI et CMF en pourcentage (P/P) et en mg/L pour les produits seuls et l'association.

La concentration maximale d'essai correspond à la concentration de la solution mère /2. Tableau 1

[Table 1]

| M. globosa | CMI | | CMF | |
|---|---|---|---|---|
| | % | mg/L | % | mg/L |
| PO | $2,3 \times 10^{-3}$ | 23 | $2,3 \times 10^{-3}$ | 23 |
| CPO | $2,9 \times 10^{-3}$ | 29 | $2,9 \times 10^{-3}$ | 29 |
| PO / CPO | $7,32 \times 10^{-5} / 3,65 \times 10^{-4}$ | 0,732/3,65 | $7,32 \times 10^{-5} / 3,65 \times 10^{-4}$ | 0,732/3,65 |
| Kéto | | 0,98 | | 1,94 |
| PO : Piroctone Olamine ; CPO : Ciclopiroxolamine ; Kéto : kétoconazole | | | | |

Tableau 2

[Table 2]

| M. restricta | CMI | | CMF | |
|---|---|---|---|---|
| | % | mg/L | % | mg/L |
| PO | $2,3 \times 10^{-3}$ | 23 | $2,3 \times 10^{-3}$ | 23 |
| CPO | $1,5 \times 10^{-3}$ | 15 | $1,5 \times 10^{-3}$ | 15 |
| PO / CPO | $7,32 \times 10^{-5} / 3,65 \times 10^{-4}$ | 0,732/3,65 | $7,32 \times 10^{-5} / 3,65 \times 10^{-4}$ | 0,732/3,65 |
| Kéto | | 0,49 | | 0,49-0,98 |
| PO : Piroctone Olamine ; CPO : Ciclopiroxolamine ; Kéto : kétoconazole | | | | |

Il n'a pas été noté d'activité pour l'éthanol dans les conditions d'essai.

Les tableaux 1 et 2 montrent que la Piroctone Olamine et la Ciclopiroxolamine seules exercent une forte activité inhibitrice, tout commecelle du kétoconazole qui sert de témoin positif. Le résultat le plus significatif est trouvé avec l'association des deux produits, en effet, un effet inhibiteur synergique est mis en évidence avec une réduction des CMI d'un facteur 10 à 100 comparativement aux CMI des produits seuls. Les résultats entre les deux souches testées sont tout à fait comparables.

Une synergie entre la Piroctone Olamine et la Ciclopiroxolamine est en effet très clairement démontrée lorsque l'on calcule l'indice de synergie qui atteint seulement 0,16 sur *Malassezia globosa* et 0,28 sur *Malassezia restricta.*

Cette synergie entre la Piroctone Olamine et la Ciclopiroxolamine notamment sur *M. globosa* permet également de démontrer un effet bénéfique dans la dermatite séborrhéique.

Exemple 2 : Effets de l'association de la Ciclopiroxolamine et de la Piroctone Olamine sur l'activité *anti-Malassezia*

La capacité de la Piroctone Olamine et de la Ciclopiroxolamine à inhiber la croissance des colonies de *Malassezia* est importante mais est insuffisant pour prédire d'une activité létale de *Malassezia* et par conséquent d'une élimination optimale des pellicules. En effet, le *Malassezia* peut à nouveau proliférer s'il n'est pas totalement ou suffisamment éliminé. Par conséquent, l'efficacité d'un actif pelliculaire doit également être mise en évidence par la démonstration de son effet sur la létalité de *Malassezia* cette composante est généralement mesurée par l'estimation de décroissance des colonies microbiennes au cours du temps.

Dans le cadre de ce travail, le modèle a été optimisé en termes de préparation des échantillons, du temps de contact et de concentration d'évaluation des échantillons.

Le but du test décrit ci-dessous est d'évaluer de manière pertinente l'activité anti-*Malassezia* (*M. globosa* et *M. restricta*) de la Piroctone Olamine, de la Ciclopiroxolamine et de leur combinaison et de la comparer avec celle du Kétoconazole.

Les produits à tester seuls et associés aux concentrations définies sont mis en contact avec une suspension de *Malassezia* titrant $10^7$ UFC/ml durant les temps de contact de 5 min, 15 min et 30 minutes. Après chaque temps de contact, un échantillon est prélevé puis dilué et filtré sur membrane de cellulose. Chaque membrane est ensuite déposée sur le milieu gélosé (Dixon) et incubé à 30°C. Après incubation, les colonies sont dénombrées.

Les produits testés sont :

- La Ciclopiroxolamine, à 1,5% (P/P) diluée avec de l'eau distillée stérile,
- La Piroctone Olamine à 0,3% (P/P) solubilisé dans de l'éthanol, puis ajout d'eau pour préparations injectables (concentration finale en éthanol à 9%)
- Le Ketoconazole (témoin positif) à 2% (P/P) solubilisé dans du DMSO, puis ajout d'eau pour préparations injectables (concentration finale en DMSO 9%).
- L'association Piroctone Olamine / Ciclopiroxolamine, aux concentrations respectivement de 0,3% et 1,5%.

Les solutions sont préparées au moment de l'essai et utilisées immédiatement.

Résultats

Les résultats obtenus avec ces différents produits sont présentés dans les tableaux 3 (*Malassezia globosa*) et 4 (*Malassezia restricta*) ci-dessous.

Tableau 3 : Fongicidie des actifs sur *Malassezia globosa*

[Table 3]

|  | Log Réduction avec différents temps de contact | | |
|---|---|---|---|
|  | 5 min | 15 min | 30 min |
| Piroctone Olamine (PO) | 0,36 | 0,61 | 1,24 |
| Ciclopiroxolamine (CPO) | 2,45 | 3,88 | >4,22 |
| PO / CPO | >4,27 | >4,27 | >4,27 |
| Kétoconazole | 1,47 | 1,29 | 1,19 |

Tableau 4 : Fongicidie des actifs sur *Malassezia restricta*

[Table 4]

|  | Log Réduction avec différents temps de contact | | |
|---|---|---|---|
|  | 5 min | 15 min | 30 min |
| Piroctone Olamine (PO) | 1,19 | 1,28 | 1,73 |
| Ciclopiroxolamine (CPO) | 1,99 | 3,3 | >4,17 |
| PO / CPO | >4,44 | >4,44 | >4,44 |
| Kétoconazole | 0,85 | 1,12 | 1,09 |

Le kétoconazole présente une activité sur *Malassezia globosa* et *restricta* aux trois temps de contact examinés, cette activité bien que modeste permet de valider ce test.

La Piroctone Olamine et la Ciclopiroxolamine montrent une activité anti-*Malassezia* supérieure à celle trouvée avec le Kétoconazole. De façon surprenante, la combinaison de la Piroctone Olamine avec la Ciclopiroxolamine se révèle encore plus efficace, notamment au temps de contact de 5 minutes. En effet, cette association permet d'obtenir un pourcentage de réduction du nombre de cellules viables de Mallassezia globosa ou Malassezia restricta supérieure à 99,99%, dès 5 minutes de contact. Les inventeurs démontrent ainsi une synergie entre la Piroctone Olamine et la Ciclopiroxolamine sur les deux souches testées. La combinaison de ces deux actifs présente donc une activité antipelliculaire très intéressante, par son niveau d'efficacité mais également par sa rapidité d'action ce qui présente un net effet avantageux permettant un temps de pose réduit et raisonnable dans le cadre de traitement antipelliculaire.

**Revendications**

1. Association comprenant de la Ciclopiroxolamine et de la Piroctone Olamine, sans pyrithione de zinc pour son utilisation pour prévenir et/ou traiter la dermatite séborrhéique et/ou pour combattre les pellicules et/ou en prévenir la formation.

2. Association pour utilisation selon la revendication 1, **caractérisée en ce que** l'association est sous une forme adaptée pour être administrée par voie topique.

3. Composition comprenant à titre de principe actif une association comprenant de la Ciclopiroxolamine et de la Piroctone Olamine, sans pyrithione de zinc avec au moins un excipient dermatologiquement ou cosmétiquement acceptable pour son utilisation pour prévenir et/ou traiter la dermatite séborrhéique et/ou pour combattre les pellicules et/ou en prévenir la formation.

4. Composition pour son utilisation selon la revendication 3, **caractérisée en ce qu'**elle comprend 0,01 à 2% en poids de Ciclopiroxolamine par rapport au poids total de la composition.

5. Composition pour son utilisation selon l'une des revendications 3 ou 4, **caractérisée en ce qu'**elle comprend 0,01 à 1% en poids de Piroctone Olamine par rapport au poids total de la composition.

6. Composition pour son utilisation selon l'une quelconque des revendications 3 à 5, **caractérisée en ce que** la composition est sous une forme adaptée pour être administrée par voie topique.

7. Composition pour son utilisation selon l'une quelconque des revendications 3 à 6, **caractérisée en ce qu'**elle comprend en outre un autre principe actif antipelliculaire.

8. Méthode de traitement cosmétique pour combattre les pellicules et/ou en prévenir la formation comprenant l'administration, notamment par application topique au niveau des cheveux ou du cuir chevelu, d'une association telle que décrite dans la revendication 1 ou d'une composition telle que décrite dans l'une des revendications 3 à 7.

9. Procédé permettant d'obtenir une efficacité antipelliculaire sur cheveux ou cuir chevelu comprenant les étapes consistant à - (i) mouiller les cheveux avec de l'eau, - (ii) appliquer une quantité efficace d'une composition antipelliculaire telle que décrite dans l'une des revendications 3 à 7 sur les cheveux, - (iii) rincer la composition antipelliculaire des cheveux avec de l'eau, - (iv) éventuellement répéter les étapes (ii) et (iii).

10. Utilisation d'une association selon l'une des revendications 1 ou 2 pour la fabrication d'une composition dermo-cosmétique pour combattre les pellicules et/ou en prévenir la formation.

**Patentansprüche**

1. Kombination, umfassend Ciclopiroxolamin und Pirocton-Olamin, ohne Zinkpyrithion für ihre Verwendung zur Vorbeugung und/oder Behandlung der seborrhoischen Dermatitis und/oder zur Bekämpfung der Schuppen und/oder zur Vorbeugung von deren Bildung.

2. Kombination zur Verwendung nach Anspruch 1, **dadurch gekennzeichnet, dass** die Kombination in einer Form vorliegt, die zur Verabreichung auf topischem Weg geeignet ist.

3. Zusammensetzung, umfassend als Wirkstoff eine Kombination, die Ciclopiroxolamin und Pirocton-Olamin umfasst, ohne Zinkpyrithion mit mindestens einem dematologisch oder kosmetisch akzeptablen Hilfsstoff für ihre Verwendung zur Vorbeugung und/oder Behandlung der seborrhoischen Dermatitis und/oder zur Bekämpfung der Schuppen und/oder zur Vorbeugung von deren Bildung.

4. Zusammensetzung für ihre Verwendung nach Anspruch 3, **dadurch gekennzeichnet, dass** sie 0,01 bis 2 Gew.-% Ciclopiroxolamin im Verhältnis zum Gesamtgewicht der Zusammensetzung umfasst.

5. Zusammensetzung für ihre Verwendung nach einem der Ansprüche 3 oder 4, **dadurch gekennzeichnet, dass** sie 0,01 bis 1 Gew.-% Pirocton-Olamin im Verhältnis zum Gesamtgewicht der Zusammensetzung umfasst.

**6.** Zusammensetzung für ihre Verwendung nach einem der Ansprüche 3 bis 5, **dadurch gekennzeichnet, dass** die Zusammensetzung in einer Form vorliegt, die die zur Verabreichung auf topischem Weg geeignet ist.

**7.** Zusammensetzung für ihre Verwendung nach einem der Ansprüche 3 bis 6, **dadurch gekennzeichnet, dass** sie ferner einen anderen Anti-Schuppen-Wirkstoff umfasst.

**8.** Kosmetische Behandlungsmethode zur Bekämpfung der Schuppen und/oder zur Vorbeugung von deren Bildung, umfassend die Verabreichung, insbesondere durch topische Anwendung im Bereich des Haars oder der Kopfhaut, einer Kombination nach Anspruch 1 oder einer Zusammensetzung nach einem der Ansprüche 3 bis 7.

**9.** Verfahren, das erlaubt, eine Anti-Schuppen-Wirkung auf dem Haar oder der Kopfhaut zu erhalten, umfassend die folgenden Schritte - (i) Nassmachen des Haars mit Wasser, - (ii) Auftragen einer wirksamen Menge einer Anti-Schuppen-Zusammensetzung nach einem der Ansprüche 3 bis 7 auf das Haar, - (iii) Ausspülen der Anti-Schuppen-Zusammensetzung aus dem Haar mit Wasser, - (iv) gegebenenfalls die Schritte (ii) und (iii) wiederholen.

**10.** Verwendung einer Kombination nach einem der Ansprüche 1 oder 2 zur Herstellung einer dermokosmetischen Zusammensetzung zur Bekämpfung der Schuppen und/oder zur Vorbeugung von deren Bildung.

**Claims**

**1.** A combination comprising Ciclopiroxolamine and Piroctone Olamine, without zinc pyrithione, for its use to prevent and/or treat seborrheic dermatitis and/or to combat dandruff and/or prevent its formation.

**2.** The combination for its use according to claim 1, **characterized in that** the combination is in a form suitable for being administered topically.

**3.** A composition comprising as active ingredient a combination comprising Ciclopiroxolamine and Piroctone Olamine, without zinc pyrithione with at least one dermatologically or cosmetically acceptable excipient for its use to prevent and/or treat seborrheic dermatitis and/or to combat dandruff and/or prevent its formation.

**4.** The composition for its use according to claim 3, **characterized in that** it comprises 0.01 to 2% by weight of Ciclopiroxolamine relative to the total weight of the composition.

**5.** The composition for its use according to one of claims 3 or 4, **characterized in that** it comprises 0.01 to 1% by weight of Piroctone Olamine relative to the total weight of the composition.

**6.** The composition for its use according to any one of claims 3 to 5, **characterized in that** the composition is in a form suitable for being administered topically.

**7.** The composition for its use according to any one of claims 3 to 6, **characterized in that** it further comprises another anti-dandruff active ingredient.

**8.** A cosmetic treatment method for combating dandruff and/or preventing its formation comprising administering, in particular by topical application to the hair or scalp, a combination as described in claim 1 or a composition as described in one of claims 3 to 7.

**9.** A process for obtaining anti-dandruff effectiveness on hair or scalp comprising the steps consisting of - (i) wetting the hair with water, - (ii) applying an effective amount of an anti-dandruff composition as described in one of claims 3 to 7 on the hair, - (iii) rinsing the anti-dandruff composition from the hair with water, - (iv) optionally repeating steps (ii) and (iii).

**10.** Use of a combination according to any one of claims 1 or 2 for the manufacture of a dermo-cosmetic composition to combat dandruff and/or prevent its formation.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **TAJIMA et al.** *J Invest Dermatol,* 2008, vol. 128, 345-351
- **PIÉRARD-FRANCHIMONT et al.** *Dermatology,* 2001, vol. 202, 171-176
- **DEANGELIS et al.** *J Invest Dermatol,* 2007, vol. 127, 2138-2146
- *CHEMICAL ABSTRACTS,* 68890-66-4
- *CHEMICAL ABSTRACTS,* 29342-05-0